# EUROPEAN PATENT SPECIFICATION

(11) **EP 3 950 028 B1**
(45) Date of publication and mention of the grant of the patent: **07.08.2024**
(21) Application number: 21171539.6
(22) Date of filing: 17.08.2011
(51) Int. Cl.: A61M 11/00, A61M 11/04, A61M 11/06, A61M 15/00, A61M 16/00, A61M 16/10, A61M 16/20

(54) **SYSTEMS OF AEROSOL DELIVERY WITH AIRFLOW REGULATION**
SYSTEME ZUR AEROSOLZUFUHR MIT LUFTFLUSSREGULIERUNG
SYSTÈMES D'ADMINISTRATION D'UN AÉROSOL AVEC RÉGULATION DU FLUX D'AIR

(30) Priority: 23.08.2010 US 80687410
(43) Date of publication of application: 09.02.2022
(62) Divisional of application: 11820272.0
(73) Proprietor: Rubin, Darren, Largo, FL 33774 (US)
(72) Inventor: Rubin, Darren, Largo, FL 33774 (US)
(74) Representative: Lloyd, Robin Jonathan

(56) References cited:
- CN-U- 201 509 598
- US-A1- 2007 102 013
- US-A1- 2009 126 745
- US-A1- 2009 151 718

## Description

### BACKGROUND OF THE INVENTION

The present invention relates to an aerosol delivery device for providing controlled airflow through the device to optimize aerosol delivery under a greater range of conditions.

### Description of the Prior Art

The term "aerosol" is understood in the context of the present invention to mean a preferably nebulous collection of atomized liquid droplets or fine powder particles that can be available for inhalation. The term "aerosolized therapy" as used herein means any aerosolized liquid or powder, or the condensation aerosol that forms after vaporization of a substance, regardless of whether it is physiologically active. The expression "medicament formulation" used in the present invention is understood to include, apart from medicaments, also therapeutic agents or the like, in particular therefore all types of agents for inhalation, including those which are active and non-active ingredients.

In most instances, aerosol particles with a mass median aerodynamic diameter, MMAD, between 0.5 and 5 micrometers are ideal for lung delivery; whereas, aerosol particles with a MMAD of greater than 5 micrometers have deposition in the upper airways rather than the lungs. Aerosol particles with a MMAD of 2 to 5 micrometers have deposition in the bronchi and bronchioles, and aerosol particles with a MMAD of less than 2 micrometers have deposition in the alveoli, for deep lung and/or systemic delivery.

The use of aerosol delivery devices of known designs and configurations, including nebulizers, vaporizers, and other inhalers, is known in the prior art.

More specifically, aerosol delivery devices of known designs and configurations previously devised and utilized for the purpose of administering medicament dosages through conventional methods and apparatuses are known to consist basically of familiar, expected, and obvious structural configurations, notwithstanding the myriad of designs encompassed by the crowded prior art which has been developed for the fulfillment of countless objectives and requirements. Such aerosol delivery devices make it possible to introduce substances to the respiratory system generally via simple inhalation.

By way of example International Patent Application and WO 03/047763 A1, European Patent Applications EP 0 471 323 A1 and EP 0 653 218 A1 and U.S. Pat. No. 5,241,954 and 7,559,491 disclose an air jet nebulizer that passes a stream of pressurized air into a liquid reservoir, which forces the liquid onto a baffle to effect aerosol generation. U.S. Pat. Nos. 3,989,042 and 7,472,701 disclose an ultrasonic nebulizer, which utilizes a piezoelectric motor or piezo-oscillating element that vibrates at ultrasonic frequencies, to pass liquid through a vibratable aperture mesh or membrane, to effect aerosol generation. Some nebulizers can be hand-held and portable, as they are battery operated, and sometimes, rechargeable, as described in U.S. Pat. Nos. 6,637,430 and 7,600,511. Nebulizers can be used with spacers and holding chambers. Nebulizers can also be fitted with adapters to provide positive expiratory pressure therapy, and/or positive airway pressure therapy, such as those disclosed by U.S. Pat. Nos. 6,253,766; 6,904,906; and 7,191,780.

Nebulizers may also conserve medication by incorporated a pump that is breath-activated, and may be turned on and off depending on the stage in the patient's breathing cycle. The breathing cycle includes the stages of inhalation, pause, and exhalation. For instance, U.S. Pat. No. 5,894,841 describes a pressure transducer, responsive to inhalation, that may activate the pump during inhalation, and inactivate the pump when inhalation is no longer detected, i.e., during exhalation, or with a timer. Likewise, U.S. Pat. Nos. 7,131,439 and 7,634,995 describe a breath activated nebulizer, with a jet that becomes active during inhalation.

Other means to aerosolize a liquid, without the use of compressed air or a piezoelectric motor, include U.S. Pat. No. 7,621,266; which describes a liquid reservoir whose contents are forced through one or more nozzles under pressure, by mechanical means, to generate aerosol for delivery. The velocity of the emitted droplet stream may be slowed with nozzles angled toward one another.

Electricity can also be used to generate aerosol by vaporizing a medicament formulation with heat from an electrically resistive heating element, electrothermal transducer, or thermo-electrical converter, and allowing that vaporized substance to condense or react in the airflow of the device, as described in U.S. Pat. Nos. 5,881,716 and 7,540,286. Electricity used to power a vaporizer may also be generated from a micro power source, such as a micro-fuel cell, as described in U.S. Pat. No. 7,665,460. More information about fuel cells are revealed by U.S. Pat. Nos. 7,059,582, 7,329,348 and 7,655,331. Whereas, U.S. Pat. No. 7,581,540 discloses a vaporizer that uses heat generated by the ignition of a fuel.

Unlike nebulizers, metered dose inhalers, MDI, generally consist of a canister filled with a liquefied gas propellant, stabilizing excipients, and medicament. The canister contains a metering valve that dispenses into a discharge nozzle within the inhaler. U.S. Pat. Nos. 3,732,864; 4,291,688; 7,597,098; and 7,600,512 describe metered dose inhalers, some of which are self-actuated by patient breath, and include a dosage counter. Some MDIs contain a spacer region, as disclosed by U.S. Pat. Nos. 5,178,138 and 6,718,969; while other MDIs attach to a separate holding chamber, as disclosed by U.S. Pat. No. 6,240,917. Spacers and holding chambers can come in many different sizes and shapes, such as the conical shape disclosed in U.S. Pat. No. 5,178,138, and may include spiral or impeller-like baffles to generate a rotational flow of aerosolized air, as disclosed in U.S. Pat. Nos. 5,309,900 and 5,596,982 and 7,562,656; and may be made of an electrostatically neutral material, or contain an anti-static coating as disclosed in U.S. Pat. No. 7,562,656, to avoid attraction and impaction of aerosol droplets with the device.

Medicament is not limited to a liquid format. Solid particles can also be inhaled as a fine powder, without a propellant, if they are dispersed into an airflow stream using a dry powder inhaler, DPI, such as disclosed in U.S. Pat. Nos. 4,524,769 and 7,624,733. Dry powder inhalers may also use a vibratable plate to disaggregate solid medicament particles, as described in U.S. Pat. No. 7,334,577.

The supply of a fluid fed to an aerosol generator can be controlled as disclosed in U.S. Pat. Nos. 7,628,339 and 7,360,536 to affect dosing of a medicament. Electronic means can be employed to achieve such control.

In aerosol delivery devices, valves, such as duckbill valves and flapper valves, a flexible valve that bends in response to a pressure differential, can be employed to allow aerosol to reach the patient only during inhalation, as to reduce aerosol loss. Such valves may also be employed to prevent backflow of a patient's expired air into the device. Additionally, filters may be employed to reduce exposure by caregivers of contaminated patient air and aerosol. Such valves and filters are described by U.S. Pat. Nos. 7,571,722; 7,204,245; and 6,904,906.

Most aerosol delivery occurs through the mouth, but nasal delivery of aerosol is also possible. U.S. Pat. No. 7,347,201 describes such nasal delivery devices, which utilize a nosepiece, instead of a mouthpiece end. Face masks are also commonly used with aerosol delivery devices, as described by U.S. Pat. No. 7,082,947. Some aerosol delivery devices can also be placed in a respiratory circuit to provide aerosols to patients on mechanical ventilation, as described in U.S. Pat. No. 5,178,138, among others.

As there is a myriad of ways to generate aerosol, there is also a myriad of ways to store the medicament formulation, including liquid reservoirs, pressurized canisters, as well as in blister strips or dosage packets, as described in U.S. Pat. No. 7,334,577, and in cassettes or cartridges, as described in U.S. Pat. No. 7,540,286.

There exist numerous other ways to attempt to enhance aerosol delivery efficiency. Aerosols can be warmed to reduce particle size, as disclosed by U.S. Pat. No. 6,131,570. Aerosols can be released at a specific point in the breathing cycle, as inspiratory flow rate and inspiratory volume are detected by sensors and computed by microprocessors, as disclosed by U.S. Pat. No. 6,250,298.

Some respiratory devices may measure or indicate airflow. U.S. Pat. No. 6,656,129 describes a flow based incentive spirometer. U.S. Pat. No. 6,679,250 describes a combination spirometer or peak flow meter and nebulizer system to measure flow rate of breath exhaled during the exhale phase. U.S. Pat. Nos. 6,904,908 and 7,201,165 describe a flow/visual indicator for an aerosol medication delivery system. U.S. Pat. No. 6,955,169 describes an inhaler device with a float to show airflow.

U.S. Pat. No. 7,073,499 describes an inhaler with airflow regulation that is limited in scope to the involuntary regulation of an airflow passage by the force of inhaled airflow, and cannot be adjusted by other means; such as by manual adjustment by hand or by electro-mechanical, motor, means. Therefore, the involuntary airflow regulation, and thus airflow rate, of the device disclosed by 7,073,499 is constant and not controllable, and provides a limited range of airflow resistance that must be commensurate with the user's inspiratory rate. The threshold of the device cannot be adjusted. U.S. Pat. No. 7,185,651 describes a dry powder inhaler with a threshold valve and a flow regulating valve that allows actuation of the device. However, both the threshold valve and flow regulating valve are non-adjustable, and only allow for a very limited range of airflow. Likewise, U.S. Pat. No. 6,655,379 also describes a device with a non-adjustable, flow restrictor valve that limits flow rates to less than 17 liters per minute.

U.S. Pat. No. 6,606,992 relates to techniques for regulating the flow of inspired gases when dispersing a pharmaceutical formulation. More specifically, this system relates to the aerosolization of pharmaceutical formulations using energy created by patient inhalation, to synchronize aerosol generation with inhalation, after a threshold vacuum is exceeded. In this case, inspired gases are used to disperse and deagglomerate a powdered pharmaceutical formulation for deep lung delivery. This device is very limited in means to generate aerosols. The major flaw of this system is that there are no calibrated airflow resistance settings, so that if a restriction mechanism is adjusted, there is no way of knowing what the resulting airflow rate will be, without measuring the airflow of the device with laboratory instruments, each and every time the device is altered. As such, the airflow rate may be adjusted incorrectly by users and care givers to produce a less than desirable outcome for aerosol delivery. Unlike the present invention, the device disclosed by U.S. Pat. No. 6,606,992 is also limited by lack of a spacer, holding chamber, reserve chamber, region so that aerosol may not have adequate time and space to disperse properly so that aerosol velocity, and/or aerosolized airflow velocity, cannot be slowed and/or controlled as effectively. Furthermore, without a spacer region, aerosol particles may not deagglomerate or evaporate as effectively, which is needed to obtain aerosols of a higher percentage of decreased particle size for improved lung delivery.

The devices disclosed by Rubin in U.S. Pat. Nos. 4,444,202; 6,539,939; 6,708,688; and 6,718969, and by Dwork in U.S. Pat. No. 5,522,380 describe respiratory therapy systems, with calibrated airflow resistance settings, that can perform both lung exercise and aerosol delivery when coupled to a nebulizer device. However, these large and complex systems have inherent limitations and are not designed to provide controlled airflow through the device to optimize aerosol delivery under a greater range of conditions. U.S. Pat. Nos. 4,444,202 and 5,522,380 are not dedicated aerosol delivery devices, themselves, but U.S. Pat. Nos. 6,539,939; 6,708,688; and 6,718969 can deliver metered dose aerosol, MDI. However, MDI inhalers are typically unable to efficiently deliver aerosol particles with a MMAD small enough for deep lung delivery, and thus cannot provide adequate systemic delivery of a therapeutic substance via the pulmonary route. Therefore, the devices specified by U.S. Pat. Nos. 4,444,202; 5,522,380; 6,539,939; 6,708,688; and 6,718969 have only a limited range of treatments options available to them. These devices perform under a limited range of conditions with a limited variety of medicaments. There exist other methods of aerosolization, such as vaporization, that can accommodate a greater variety of medicaments and formulations, that these devices cannot provide. Furthermore, these devices do not provide nasal aerosol delivery. Moreover, these devices are not self-actuating, and therefore, may be difficult to time the coordination of dispensing medicament with patient inhalation.

US 2009/126745 A1 discloses an emulation aerosol sucker.

US 2009/151718 A1 discloses apparatuses and methods for diagnosing and treating respiratory conditions.

There are numerous limitations inherent in prior aerosol delivery devices. All of these aforementioned devices are not able to provide the optimal amount of airflow regulation under all conditions of aerosol delivery. Unlike the present invention, prior aerosol delivery devices do not accomplish all of the following:
A) greater control over laminar flow and/or flow velocity and volume of aerosolized air for improved aerosol delivery to patient airways;
B) greater and longer expansion of patient airways, such as with positive pressure, so that airways are more receptive to receiving aerosolized medicament formulations;
C) selective targeting of aerosols to different regions of the airways, such as the upper airways, lower airways, and/or providing systemic delivery through the pulmonary route;
D) accommodation of the full range of varying degrees of patient lung function and/or inspiratory ability, including, but not limited to, pediatric patients with small lung volumes, chronic obstructive pulmonary disease, COPD, patients with compromised lung function, and adult patients with healthy lung function;
E) accommodation of more medicament formulations that have potential for aerosolization; including liquids and solids, droplets and particles, of varying sizes, shapes, weights, and flow dynamic properties; and therefore, prior aerosol delivery devices do not provide for enhanced efficiency of aerosol delivery under a wide range of medicament formulations, to a wide variety of patients, and to various regions of the airways, as the present invention does.
To attain this, there is provided an aerosol delivery device according to Claim 1.
There has thus been outlined, rather broadly, the more important features of the invention in order that the detailed description thereof that follows may be better understood and in order that the present contribution to the art may be better appreciated. There are, of course, additional features that will be described hereinafter and which will form the subject matter of the claims attached.

To attain this, the present invention essentially comprises a housing with at least one airflow inlet, at least one airflow outlet, and at least one airflow passage extending therebetween. A medicinal or therapeutic substance to be inhaled is provided. Within this housing is at least one site/element for producing and/or dispensing an aerosol to be entrained by airflow through the device. At least one calibrated airflow resistance control element with adjustable settings allows regulation of airflow into, through, and/or out of the invention.

In this respect, before explaining at least one embodiment of the invention in detail, it is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of descriptions and should not be regarded as limiting.

As such, those skilled in the art will appreciate that the conception, upon which this disclosure is based, may readily be utilized as a basis for the designing of other structures, methods and systems for carrying out the several purposes of the present invention. It is important, therefore, that the claims be regarded as including such equivalent constructions insofar as they do not depart from the scope of the present invention.

It is therefore an object of the present invention to provide a new and improved aerosol delivery device which has all of the advantages of the prior art aerosol delivery devices of known designs and configurations and none of the disadvantages.

It is another object of the present invention to provide a new and improved aerosol delivery device which may be easily and efficiently manufactured and marketed.

It is further object of the present invention to provide a new and improved aerosol delivery device which is of durable and reliable constructions.

An even further object of the present invention is to provide a new and improved aerosol delivery device which is susceptible of a low cost of manufacture with regard to both materials and labor, and which accordingly is then susceptible of low prices of sale to the consuming public, thereby making such aerosol delivery device economically available to the buying public.

Even still another object of the present invention is to provide an aerosol delivery device for providing controlled airflow through the device to optimize aerosol delivery under a greater range of conditions.

Lastly, it is an object of the present invention to provide a new and improved aerosol delivery device comprising a housing with at least one airflow inlet, at least one airflow outlet, and at least one airflow passage extending therebetween. A medicinal or therapeutic substance to be inhaled is provided. Within this housing is at least one site/element for producing and/or dispensing an aerosol to be entrained by airflow through the device. At least one calibrated airflow resistance control element with adjustable settings allows regulation of airflow into, through, and/or out of the invention.
These together with other objects of the invention, along with the various features of novelty which characterize the invention, are pointed out with particularity in the claims annexed to and forming a part of this disclosure. For a better understanding of the invention, its operating advantages and the specific objects attained by its uses, reference should be had to the accompanying drawings and descriptive matter in which there is illustrated preferred embodiments of the invention.

### BRIEF DESCRIPTION OF THE DRAWINGS

The invention will be better understood and objects other than those set forth above will become apparent when consideration is given to the following detailed description thereof. Such description makes reference to the annexed drawings wherein:
FIG. 1 is an improved aerosol delivery device of a first exemplary aerosol delivery device not forming part of the present invention that serves as a portable, sensor activated, ultrasonic nebulizer with a calibrated airflow resistance control element adjustable by hand, with digital inputs and display, along with filter accessory.
FIG. 2 is a second exemplary aerosol delivery device not forming part of the present invention that serves as a jet nebulizer with a compressed air inlet and a motor powered calibrated airflow resistance control element, with digital control unit, and nosepiece.
FIGS. 3A and 3B show greater detail of the jet nozzle, jacket, baffle, and nebulization chamber of the jet nebulizer described in FIG. 2.
FIGS. 4A and 4B show the airflow resistance control element, wheel, with different settings, from the back view perspective of the jet nebulizer described in FIG. 2.
FIG. 5 is another second example of an aerosol delivery device not forming part of the present invention that serves as a breath actuated jet nebulizer with a calibrated airflow resistance control element adjustable by hand, along with filter accessory.
FIG. 6 shows the breath actuated jet nebulizer described in FIG. 5 , along with an exhalation threshold resistance valve accessory.
FIG. 7 is a third example of an aerosol delivery device not forming part of the present invention that serves as a portable, sensor activated vaporizer powered by battery with electronically controlled calibrated airflow resistance control element, airflow sensors, and digital auditory and visual outputs.
FIG. 8 is another third example of an aerosol delivery device not forming part of the present invention that serves as a vaporizer powered by a fuel cell, with a manually adjustable calibrated airflow resistance control element.
FIG. 9 is an embodiment that serves as a digital vaporizer with flash drive, powered through a USB port, with a slidable calibrated airflow resistance control element.
FIG. 10 is an exploded view of the medicament SD memory card with vaporizer element to be used with the USB flash drive digital vaporizer described in FIG. 9 . Airflow is also shown as indicated by arrows.
FIG. 11 is an exploded view of the USB flash drive digital vaporizer described in FIG. 9 with airflow conduit. Airflow is also shown as indicated by arrows.

The same reference numerals refer to the same parts throughout the various Figures.

### DESCRIPTION OF THE PREFERRED EMBODIMENTS

With reference now to the drawings, the preferred embodiments of the new and improved systems and methods of aerosol delivery with airflow regulation embodying the principles and concepts of the present invention will be described in the following preferred aerosol delivery device embodiments.

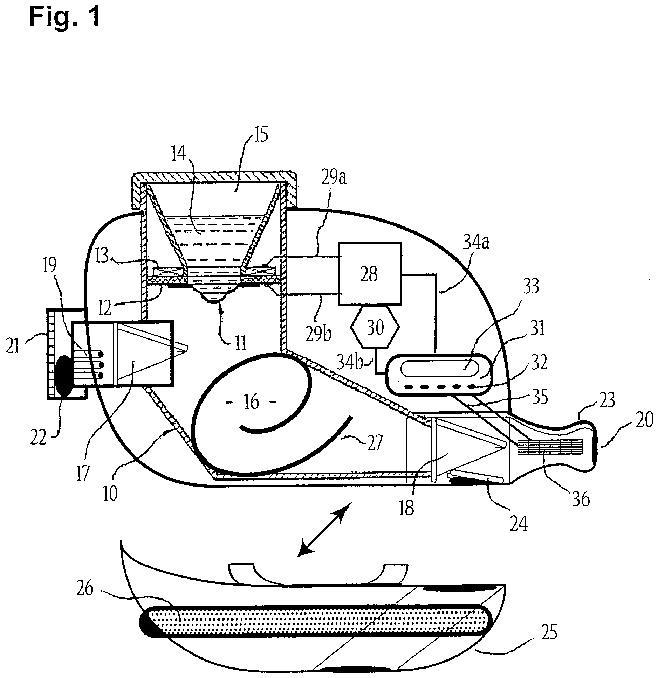

FIG. 1 shows a cross-section of an example of an aerosol delivery device 10 not according to the invention comprising a vibratable, porous membrane 11 that is caused to oscillate at a desired frequency by piezoelectric motor assembly 12,13 in response to an electric drive signal, as will later be explained. The piezoelectric motor assembly is comprised of a support unit 12 and a piezo-electrical conversion unit 13, both containing or comprised of electrically conductive material. Both support unit 12 and piezo-electrical conversion unit 13 are attached to each other, and both are attached to vibratable membrane 11.

The oscillation of vibratable membrane 11, which may include bending oscillations, causes a liquid medicament formulation 14, stored within a liquid reservoir 15, to be nebulized as this liquid is forced through small pores of membrane 11. The resulting nebulized aerosol travels into, and diffuses within, the large reserve chamber, holding chamber, 16.

One-way valves 17 and 18, preferably duckbill valves, trap the nebulized aerosol within the device until vacuum pressure, or a significant threshold vacuum pressure, generated from user inhalation, is able to open said one-way valves 17 and 18. Nebulized aerosol is thusly contained in reserve chamber 16 until airflow, originating at one or more airflow inlets 19, carries the aerosol through the device and out to the end user through the airflow outlet end 20 of the device.

Calibrated airflow resistance control element 21, in this example, consists of a user controlled airflow resistance dial with one or more supplemental apertures 22. The user controlled airflow resistance dial 21 is flush with the airflow inlet end of the device. Rotation of dial 21 aligns supplemental aperture(s) 22 with one or more airflow inlet passages 19, thereby controlling the amount of airflow allowed to enter the device and travel through these passages 19, having the affect of controlling the velocity and/or volume of airflow through the device. The airflow resistance settings of this device may also provide an auditory signal to the user, such as a whistle sound caused by air passing through the airflow control element.

Furthermore, the pitch of this whistle sound may vary between different airflow resistance settings and may allow the user to distinguish between such settings. Furthermore, the auditory signal may indicate for user to adjust his or her inhalation rate.

The airflow outlet end of the device may contain a mouthpiece 23 that contours to the user's lips, allowing for an airtight seal. Said mouthpiece 23 may contain an exhaust port 24, comprised of an elastomeric one-way, flap, valve, which vents user exhalation, while one-way valve 18 prevents exhalation from entering the interior of the device. An optional and/or removable filter housing assembly 25 may be aligned with exhaust port 24, to allow exhaled air to pass through a filter element 26, and out of the filter housing 25. A preferred filter element 26 may be a 3M filtrate filter, or other HEPA filter, able to capture infectious particles and aerosol particles larger than 0.3 micrometers in diameter from exhalation, thereby preventing cross contamination to nearby individuals. A contaminated filter element may be cleaned or replaced as necessary.

The interior walls of the device, such as along reserve chamber 16, may be curved and/or contain spiral baffles 27 to generate a rotational flow of aerosolized air that enters the device. Said rotational airflow may surround the aerosol and may more efficiently carry the aerosol out of the device, while reducing impaction or adhesion of aerosol with the inner walls of the device.

The device also comprises an electronic drive means 28 that sends an electric drive signal through signal lines 29a and 29b to the piezo-electrical conversion unit 13 and conductive support unit 12, of the piezoelectric motor assembly 12,13. A power source 30, preferably a rechargeable battery with an inlet for alternating current, provides the electrical energy for the electronic drive means 28. The device may further comprise a digital control unit 31, with user inputs 32, and a digital display 33, such as LCD or LED, and/or electroacoustic transducer speaker, not shown. The digital control unit 31 operates the electronic drive means 28 through circuit lines 34a and 34b. The digital control unit may also contain a microprocessor that can perform one or more functions, such as: setting the intensity of the electric drive signal, providing visual and/or auditory feedback to the user and/or health care worker, providing an alarm function to signal when a treatment is due, a timer function to measure the duration of treatment and/or to turn off operation after a certain treatment duration, a counting function to determine the number of treatments, a function to keep track of the airflow resistance settings during treatment, a time/date function to track the treatments of one or more different medicament formulations, along with any other functions obvious to the use of this device. Furthermore, the digital control unit may contain a USB port and/or memory card so that data can be interfaced with a computer or respiratory instrument.

The aerosol delivery device may also contain one or more conductivity sensing leads or panels, touch panels, 36, as an integral component of the mouthpiece, that forms a switching circuit with the digital control unit 31 via circuit leads 35. Conductivity sensing touch panels 36 receive bioelectricity through a living being in contact with the touch panel to complete this switching circuit, which may signal the digital control unit 31 to activate electronic drive means 28 so that the device may generate or dispense aerosol only when the user is able to receive such aerosol delivery. Said touch panels may, therefore, prevent aerosol loss when the user is not able to receive aerosol. The switching circuit may include one or more resistors, transistors, grounds, capacitors, and/or any other circuit components necessary for the function of this circuit. Touch panels 36 may also or instead be pressure sensing panels that detect user contact with the device. Alternatively, airflow sensors could be used in place of, or in addition to, touch panels 36. Likewise, airflow sensors would detect and/or monitor user inhalation and provide such information to the digital control unit 31 that can interpret the data so as to activate and/or regulate aerosol generation via electronic drive means 28, and/or to provide visual and/or auditory feedback to the user and/or health care worker.

In an alternative example, airflow sensors may also provide feedback of airflow and/or breathing pattern data to a digital control unit, or microprocessor, 31, which can interpret the data and can adjust airflow resistance by sending an electronic signal to an electric motor controlling a calibrated airflow resistance control element, such as that described in the next figure.

In other examples, the piezoelectric motor assembly may also serve as, or include, or be accompanied by, or be replaced by, a heat generating means to raise the temperature of the air and/or aerosolized liquid droplets within the device to promote reduced particle size and convection. Electrical resistance preferably provides the heat energy for the heat generating means, and so the heat generating element is foremost an electrically resistive heating element. Furthermore, this heat generating element may serve as a vaporizing element to vaporize a liquid into a condensation aerosol available for inhalation, and may be used with, or instead of, ultrasonic nebulization.

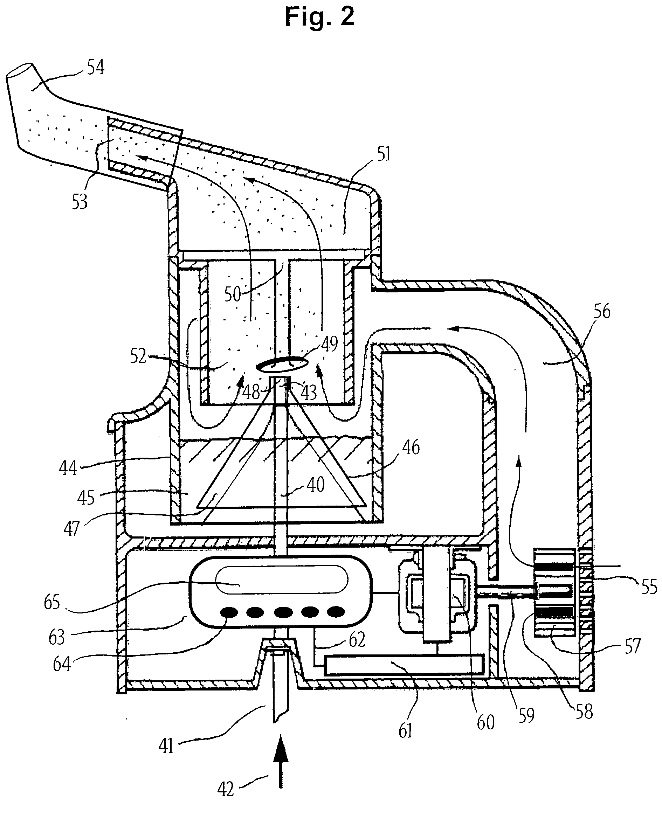

FIG. 2 shows a cross-section of a second example of an aerosol delivery device comprising a jet nozzle 40 able to receive compressed air and/or compressed oxygen from compressed gas inlet 41 connected to a source of compressed gas 42. Sources of compressed gas can may include air pumps, portable air compressors, oxygen concentrators, or pressurized medical gas tanks. The source of compressed gas 42 may even be a component of the device itself, such as if the source of compressed gas is a miniature, battery powered, air compressor. Jet nozzle 40 includes a tapered air outlet 43. The jet nozzle 40 resides inside of a liquid reservoir container 44, filled with a liquid medicament formulation 45. A jacket 46 is sleeved around the jet nozzle to define a fluid-introducing gap 47 therebetween. At the top of the jacket is a restricted opening 48. When in use, a high-pressure air jet passes through jet nozzle 40 and out through the tapered air outlet 43, causing liquid 45 to flow into the fluid-introducing gap 47 due to negative pressure generated therein. Liquid becomes nebulized into aerosol as high-pressure forces this liquid through the restricted opening 48 of jacket 46. Newly generated aerosol is dispersed as it comes in contact with a diffuser dispersing baffle 49 at high velocity. Baffle 49 is suspended by support 50. Support 50 has apertures or vents to allow aerosol to pass. Support 50 is housed by cap 51, which is connected detachably and securely to reservoir container 44. Container cap 51 also has a mist-discharging conduit or duct 52, and an aerosol outlet end 53. In this example, a nosepiece 54 is incorporated with the device, instead of a mouthpiece, so as to provide nasal aerosol delivery to the upper airways. The mist-discharging conduit 52 and/or container cap 51 may be enlarged to serve as a reserve chamber for aerosol.

This aerosol delivery device also contains one or more air inlet apertures 55 from which ambient air can enter into the device. Aerosol is carried out of the device with the assistance of airflow, depicted as arrows, which originates via inlet 55, and travels through duct 56 and into container 44, and out the aerosol outlet end 53 of container cap 51, via conduit 52, when a patient applies vacuum pressure from inhalation. The airflow that enters the device can be restricted, such as with a calibrated airflow resistance control element 57, so as to control the velocity and/or volume of airflow and/or aerosolized air through the device and out to the end user. In this example, a calibrated airflow resistance control element 57 consists of a rotatable wheel that is flush against the wall of the device in the area where inlet apertures 55 reside. The rotatable wheel 57 has a plurality of airway passages 58 which can differentially align with airway inlet(s) 55. Airflow and velocity through the device is increased when more of these airway passages 58 align with airway inlet(s) 55, and airflow and velocity through the device is decreased when less of these airway passages 58 align with airway inlet(s) 55. Axel 59 connects the rotatable wheel 57 to an electric motor 60, which uses electrical energy supplied by power source 61 to produce the mechanical energy to turn the rotatable wheel 57. A circuit 62 containing an analog or digital control unit 63, with user inputs 64, and an LCD or LED display 65 and/or electroacoustic transducer, speaker, not shown, and can operate the electric motor 60, such as to rotate the wheel 57 to the desired calibrated resistance setting, from a selection of such settings. The control unit 63 may also contain a microprocessor that can perform one or more functions, such as: providing visual and/or auditory feedback to the user and/or health care worker, providing an alarm function to signal when a treatment is due, a timer function to measure the duration of treatment and/or to signal after a certain treatment duration is complete, a counting function to determine the number of treatments, a function to keep track of the airflow resistance settings during treatment, a time/date function to track the treatments of one or more different medicament formulations, along with any other functions obvious to the use of this device. Furthermore, the control unit may contain a USB port and/or memory card so that data can be interfaced with a computer or respiratory instrument.

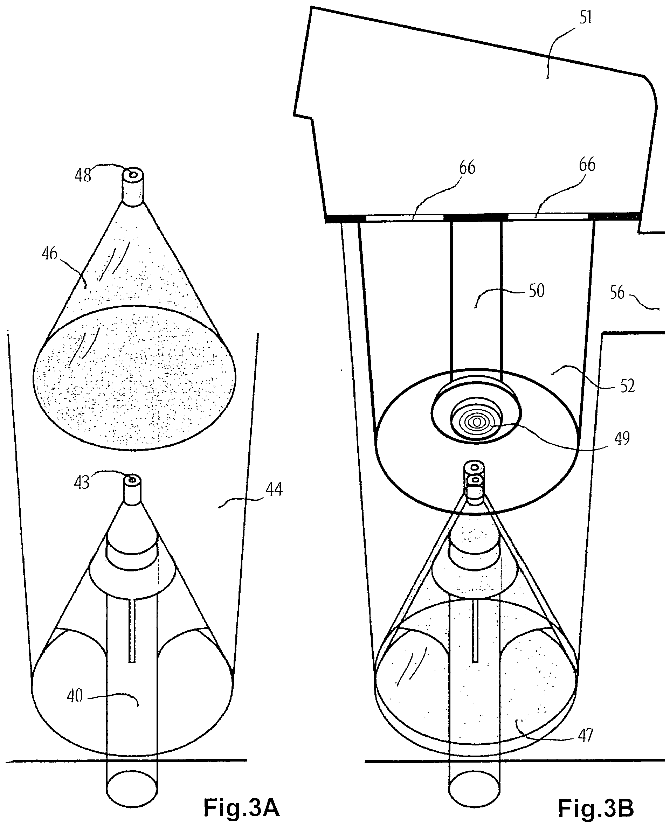

FIGS. 3A and 3B show greater detail of the jet nozzle and nebulizer container of the aerosol delivery device described in FIG. 2. FIG. 3A is an exploded view of jet nozzle 40, housed inside of liquid reservoir container 44, along with jacket 46. Jet nozzle 40 has a tapered air outlet 43, while jacket 46 has a restricted opening 48. FIG. 3B shows that when jacket 46 sleeves jet nozzle 40, a fluid-introducing gap 47 is defined therebetween, and air outlet 43 and restricted opening 48 are aligned. FIG. 3B also shows container cap 51, which detachably covers reservoir container 44, and has a mist-discharging duct 52 that extends down into the reservoir container. Within this mist-discharging duct 52 is a diffuser dispersing baffle 49, which is suspended by support 50 from the container cap. Vents 66 allow aerosol to exit through duct 52, into the container cap 51. Also shown is duct 56 where airflow from user inhalation enters the nebulizing chamber of this device.

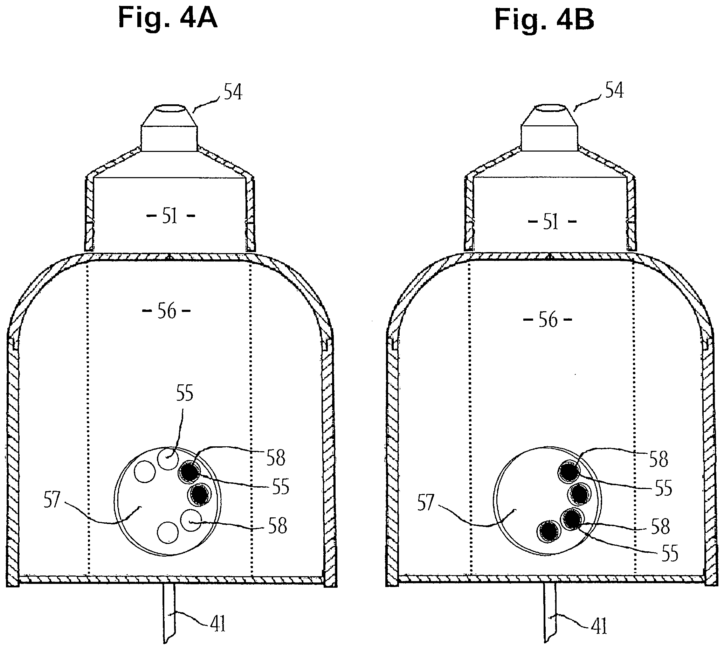

FIGS. 4A and 4B show a back view of the aerosol delivery device described in FIG. 2, along with its airflow resistance control element, wheel, 57. In this example, the housing of the device has four fixed holes that serve as air inlets 55 to air duct 56. The airflow resistance control element, wheel, 57 also has four holes that serve as airway passages 58, which can differentially align with airway inlets 55, to allow the user to draw ambient air into air duct 56 during inhalation. When few of the air passages of wheel 57 are aligned with air inlets 55, as shown in FIG. 4A , there is greater restriction to airflow than when more of the air passages of wheel 57 are aligned with air inlets 55, as shown in FIG. 4B . In this example, FIG. 4A shows the alignment of two holes, while FIG. 4B shows the alignment of all four holes. Thus, the airflow settings of FIG. 4A reduces the velocity of airflow through the device, while the airflow settings of FIG. 4B increase the velocity of airflow through the device.

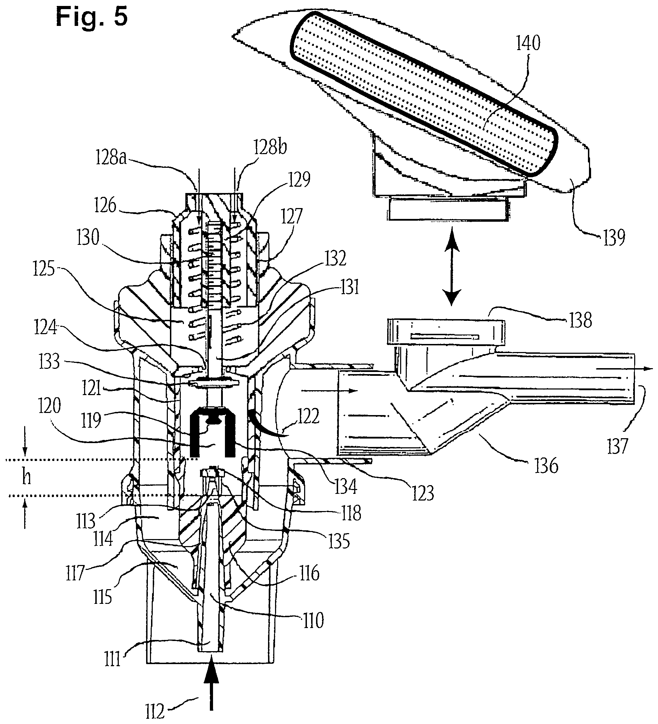

FIG. 5 shows a cross-section of another second preferred example of an aerosol delivery device comprising a jet nozzle 110 able to receive compressed air and/or compressed oxygen from compressed gas inlet 111 connected to a source of compressed gas 112. Sources of compressed gas can may include air pumps, portable air compressors, oxygen concentrators, or pressurized medical gas tanks. When the example serves as a disposable nebulizer, oxygen tubing, not shown, connects gas inlet 111 to sources of compressed gas 112. A gas flowmeter may also be connected in this gas circuit. When the example serves as a hand-held, non-disposable nebulizer, the source of compressed gas 112 may even be a component of the device itself, such as if the source of compressed gas is a miniature, battery powered, air compressor.

Jet nozzle 110 includes a tapered air outlet 113. The jet nozzle 110 resides inside of a liquid reservoir container 114, filled with a liquid medicament formulation 115. The reservoir container 114 is connected detachably and securely to the main device housing. A jacket 116 is sleeved around the jet nozzle to define a fluid-introducing gap 117 therebetween. At the top of the jacket is a restricted opening 118. When in use, a high-pressure air jet passes through jet nozzle 110 and out through the tapered air outlet 113, causing liquid 115 to flow into the fluid-introducing gap 117 due to negative pressure generated therein. Liquid becomes nebulized into aerosol as high-pressure forces this liquid through the restricted opening 118 of jacket 116. Newly generated aerosol is dispersed as it comes in contact with a diffuser dispersing baffle 119 at high velocity.

The interior chamber 120 of the device contains a conical section or chimney 121, which extends downward and surrounds the jet nozzle 110. Jacket 116 may be adjoined to chimney 121. Extension guides 122 may protrude from the walls of the chimney 121. Chimney 121 and its extension guides 122 prevent undesirable, larger droplets from exiting the device, and instead, cause such droplets to condense and return to the liquid reservoir 114. In this manner, smaller particles with a mass median aerodynamic diameter, MMAD, ideal for inhalation are able to freely exit the device through air outlet 123. Furthermore, chimney 121 and its extension guides 122 cause ambient air entering the device to take a more tortuous flow path through device to ensure that an adequate amount of aerosol is entrained in this airflow to reduce particle size and/or to prevent particles from colliding and growing. Extension guides 122 of the chimney 121, or other baffles residing in the device, may be curved or spiral-shaped to cause cyclonic action of aerosol entrained airflow. Ambient air enters interior chamber 120 through a central aperture 124 located at the top of chimney 121. Airflow through this central aperture 124 is regulated by a inhalation threshold resistance valve assembly 125. Threshold valve assembly is comprised of a rotatable cap 126 with an integrally formed cylindrical wall slidably received through the cylindrical upper region of the device housing 127. One or more air inlet ports, shown as 128a and 128b, are found at the top of rotatable cap 126. The rotatable cap also has a tubular guide 129 extending through it. The tubular guide has female threads 130 designed to receive the male threads of a thin rod 131. A load calibrated, coiled spring 132, or other resilient or biasing member, is positioned inside of the rotatable cap 126, around the tubular guide 129 and thin rod 131. A circular disc 133, along thin rod 131, is located within the interior chamber 120 of the device, thereby serving as the actuator piston of threshold valve 125. As spring 132 puts upward pressure on rotatable cap 126, circular disc 133 is pulled against the top surface of inner chamber 120, or chimney 121, and thus, blocks central aperture 124.

During inhalation, when the rate of airflow exiting the device exceeds the pressurized gas flowrate entering the device chamber, and when the negative pressure, vacuum pressure, on the valve assembly exceeds the force of the spring, the threshold valve will open as the spring compresses and the actuator piston moves down. When the valve is open, ambient air enters the device through air inlets 128, entrains nebulized particles, and carries these particles out of the device through air outlet 123. Calibrating indicia are provided on the exterior cylindrical walls of rotatable cap 126, so that threshold valve 125 also serves as a calibrated airflow resistance control element. As the cap is rotated like a dial, the distance that the thin rod 131 screws into the tubular guide 129 of the cap also changes, thereby affecting the space between the cap and the device housing 127, and thus, the compression of the spring 132. By varying the tension of the spring, one can control inhalation resistance and the velocity of airflow through the device, which may allow for aerosol delivery with sustained maximal inspiration, inhalation. Resistance settings can range from relatively minimal resistance to relatively strong resistance. One may also bypass the resistance valve at times when having no resistance is desired. This may be done by manually pushing the resistance dial down by hand, and/or twisting the dial into a locking position, which holds the resistance valve open. One may also conceive of instances where the resilient or biasing member or spring can be readily removed and replaced, and even replaced with another biasing member that has different tension properties. The device may also serve as an incentive device because movement of the threshold resistance valve assembly from inhalation may provide a visual signal, and perhaps an auditory signal, to the user.

This example can also serve as a breath activated nebulizer, if so desired. A movable diverter sheath 134 can be attached to the end of thin rod 131, a portion of the rod which extends past circular disc 133. Jacket 116, which is sleeved around jet nozzle 110, can have an open region 135, which allows pressurized gas to escape without passing through restricted opening 118, and without causing nebulization. Upon a threshold level of inhalation, the actuator piston of threshold valve 125 moves downward by a distance 'h', thereby, placing diverter sheath 134, and dispersing baffle 119, into nebulizing position. Nebulization occurs when diverter sheath 134 covers open region 135 so that pressurized gas can only escape through restricted opening 118 of jacket 116. When negative, vacuum, pressure from inhalation can no longer hold the valve open, such as towards the end of inhalation, the valve closes, the piston moves upward, and the diverter sheath is no longer in a nebulizing position. In this manner, nebulization only takes place when the user is able to inhale through this device. Downward movement of the cap 126 may also signal that inhalation and nebulization is taking place. The cap 126 may also be unscrewed and removed from the rod 131, or manually pushed down and twisted to a position, which allows for continuous nebulization. One can also conceive of a breath activated nebulizer with a diverter sheath that stays down, thereby covering openings in the jacket, while in nebulizing mode, with or without inhalation, until exhalation moves the diverter sheath up to uncover openings in the jacket, while in non-nebulizing mode.

Also shown in this figure is user mouthpiece 136 that attaches to device outlet 123. Airflow passes through the mouthpiece and out through outlet 137. Said mouthpiece may contain an exhaust port 138, containing an elastomeric one-way, flap, valve, which vents user exhalation. An optional and/or removable filter housing assembly 139 may be aligned with exhaust port 138, to allow exhaled air to pass through a filter element 140, and out of the filter housing 139. A preferred filter element 140 may be a 3M filtrate filter, or other HEPA filter, able to capture infectious particles and aerosol particles larger than 0.3 micrometers in diameter from exhalation, thereby preventing cross contamination to nearby individuals. A contaminated filter element may be cleaned or replaced as necessary.

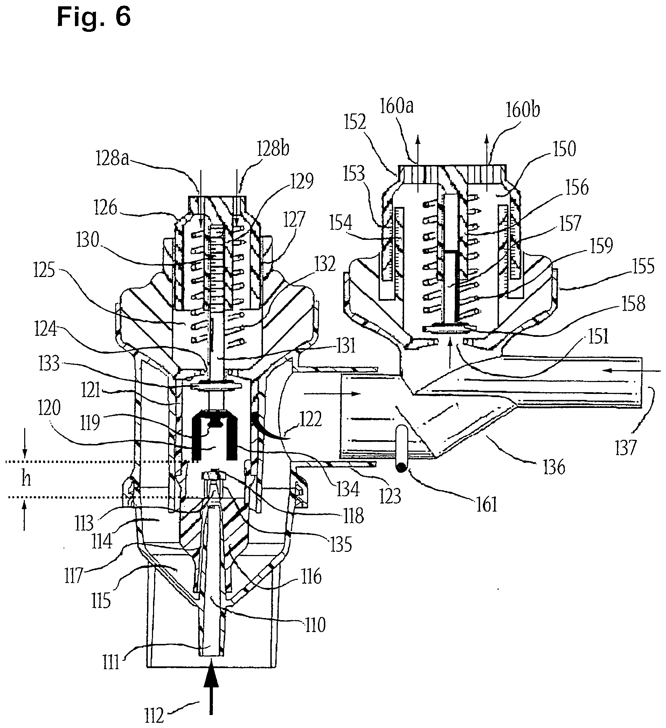

Other examples of this nebulizer may include an exhalation threshold resistance valve instead of, or in addition to, the inhalation threshold resistance valve described above. FIG. 6 shows the nebulizer depicted in FIG. 5 , but with a mouthpiece containing an exhalation threshold resistance valve that can perform positive expiratory pressure, PEP, therapy. Similarly, one can also conceive of alternative devices where the exhalation resistance valve is incorporated into the main body of the device, rather than as a component of a mouthpiece accessory. The exhalation resistance valve assembly 150 regulates airflow passing through aperture 151. The exhalation valve assembly is comprised of a rotatable cap 152 with a threaded, integrally formed cylindrical wall 153 that screws onto the upwardly extending, threaded cylindrical walls 154 of mouthpiece upper housing 155. Rotatable cap 152 also has a tubular guide 156 extending through it. The tubular guide 156 is designed to slidably receive a thin rod 157. A circular disc 158, along thin rod 157, is located above mouthpiece upper housing 155, and is able to cover aperture 151, thereby serving as the actuator piston of exhalation valve 150. A load calibrated, coiled spring 159, or other resilient or biasing member, is positioned around tubular guide 156 and thin rod 157, compressed between rotatable cap 152 and circular disc 158. One or more air outlet ports, shown as 160a and 160b, are found at the top of rotatable cap 152. Air exits through ports 160 when the exhalation vale is open. The exhalation valve opens when there is enough internal air pressure to place upward force on circular disc 158, so as to overcome the tension of the spring 159, and further compress the spring so that rod 157 can slide upwards within tubular guide 156. Disc 158 is able to move upward until it reaches, or is hindered by, tubular guide 156. Air pressure builds within the device from user exhalation through mouthpiece end 137, to open the exhalation valve. The exhalation valve closes again when air pressure decreases, such as when exhalation stops. The amount of pressure needed to open the exhalation valve can be adjusted by further screwing or unscrewing cap 152 onto cylindrical walls 154 of upper housing 155, thereby, varying the distance between cap 152 and upper housing 155. This in turn varies the compression and tension of the spring. Calibrating indicia are provided on the exterior cylindrical walls of rotatable cap 152, so that exhalation valve 150 can serve as a calibrated airflow resistance control element, as it is rotated like a dial to adjust airflow resistance settings. The device may also serve as an incentive device because movement of the threshold resistance valve assembly from exhalation may provide a visual signal, and perhaps an auditory signal, to the user.

Certain examples of this device may also use compressed air, from a first or second source, to provide positive airway pressure therapy, and may also deliver aerosol in conjunction with positive airway pressure. A Venturi may also be used to draw in ambient air, and to accelerate/decelerate air flow in the device. Positive airway pressure helps expand lungs and treat atelectasis. The positive pressure may be continuous during all portions of the breathing cycle. Or, a means of interrupting continuous positive air flow at a certain frequency, such as using a flow interrupter valve, can deliver high-frequency pulses of positive pressure, to provide for an oscillation breathing treatment, which may help clear patient airways by helping to free mucous secretions. An optional manometer, pressure, port 161 and removable cover, not shown, can allow for a manometer to accurately measure the positive pressure that the patient is receiving from the device, and may also serve as a pressure relief port. Positive airway pressure may also produce a back pressure in exhaled air to provide PEP therapy.

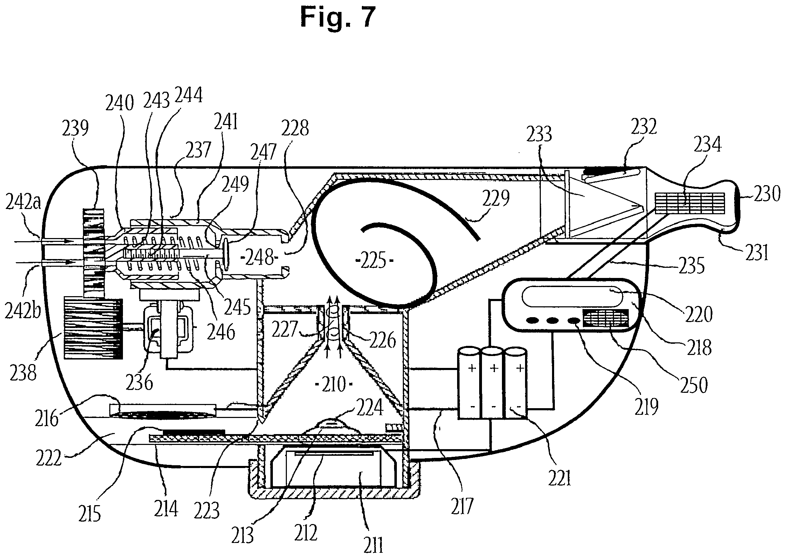

FIG. 7 shows a cross-section of a third preferred example of an aerosol delivery device comprising a horn-shaped, first chamber 210. A piezoelectric transducer 211 that is made to oscillate, vibrate, while in contact with the proximal end of first chamber 210, such as to send vibrations to that chamber 210. A heating element 212 can be comprised of an electrically resistive heating support or resistor, and is located in close communication with the proximal end of first chamber 210, such as to send heat to that first chamber, including sending heat to a medicament. The piezoelectric transducer 211 and heating element 212 may be housed together. A preformed blister pack 213, or other medicament packaging, filled with a medicament can be housed on a slidable structure, slide, strip, 214, that can be inserted into first chamber 210, such as along or near its proximal end. Slide 214 can contain a coded tag 215, such as a bar code, microchip, transmitter, radiofrequency identification tag, or other means, that can be detected and/or analyzed by an electronic tag reader 216. Tag reader 216 is able to detect the presence of the blister pack and slide. The coded information detected may also include the type of medicament and/or also its dosage and/or its serial number. The tag reader 216 may send this information through an electronic circuit 217, preferably wired to a digital control unit 218, with user inputs 219, and a digital display 220, such as LCD or LED. The digital control unit 218 controls the operation of the piezoelectric transducer 211 and heating element 212, using power from batteries 221. These batteries can either be rechargeable or non-rechargeable batteries. The detection and/or analysis of the coded medicament information 215, by the reading device 216, may allow the digital control unit 218 to turn the piezoelectric transducer 211 and heating element 212 on for certain durations, and/or may determine the desired power and frequency to operate the piezoelectric transducer 211, and may determine the desired power and temperature to heat the heating element 212, for proper delivery characteristics of that particular medicament code.

When medicament slide 214 is inserted into the aerosol delivery device, through the medicament port channel 222, a piercing means or mechanism 223 can remove or cause openings 224 on the top of blister packaging 213, by which medicament can be released into the first chamber 210. When activated, heating element 212 is able to vaporize the medicament substance from medicament slide 214 by sending thermal energy to the substance by conduction and/or convection. In other examples, heating element 212 can be located on medicament slide 214 as an electrically resistive heating support, such as a metal foil support, which may even be part of blister packaging 213. As such, medicament may be coated on this metal foil support. After vaporization, preferably with minimal degradation products of medicament, the vapor can cool and condense to form a condensation aerosol available for inhalation. As will next be described, this vapor can be efficiently carried to an aerosol holding chamber 225 where the particles can cool further.

First chamber 210 is connected to a second chamber 225 via a narrow orifice or channel 226. Vibration of the proximal end of first chamber 210 by the vibrations caused by piezoelectric transducer 211, sets up pressure variations, as well as standing waves and/or acoustic waves, within the first chamber, causing air in the first chamber 210 to move back and forth through channel 226, while vortices of air are formed at channel 226, leading to second chamber 225. A synthetic jet of air 227 is thus created by these vortices, resulting in the net flow of air from first chamber 210 into second chamber 225. Vapor and condensation aerosol is entrained in this airflow and evacuated from first chamber 210, and carried to the second chamber 225, by a synthetic jet 227 via channel 226. When the medicament substance is a dry powder, and the heating element does not vaporize some or all of the powder, such as when the heating element is not activated or when the heat transfer is less than 100% efficient, piezoelectric transducer 211 can still vibrate and mix air in the first chamber to disaggregate the dry powder released from blister pack 213, to form an aerosol. The aerosolized dry powder is entrained in the air and evacuated from first chamber 210, and carried to the second chamber 225, by a synthetic jet 227 via channel 226. As such, this aerosol delivery device can serve as a dry powder inhaler.

Second chamber 225 can serve as an aerosol reserve, holding, chamber. Airflow enters device chamber 225 through inlet passage 228, where it may be vortexed by the curved interior walls or spiral baffles 229 of this chamber, before exiting the device via outlet end 230. Airflow outlet end 230 can consist of a user mouthpiece 231 that contours to the user's lips, allowing for an airtight seal. Said mouthpiece 231 may contain an exhaust port 232, comprised of an elastomeric one-way, flap, valve, which vents user exhalation, while one-way valve 233, preferably a duckbill valve, prevents exhalation from entering the interior of the device.

The aerosol delivery device may also contain one or more airflow sensors 234, that forms a switching circuit with the digital control unit 218 via circuit leads 235. Detection of user airflow may signal the digital control unit 218 to activate and/or regulate piezoelectric transducer 211 and heating element 212 for aerosol delivery. Airflow sensors may also provide feedback of airflow and/or breathing pattern data to a digital control unit, or microprocessor, 218, which can interpret the data and can adjust airflow resistance by sending an electronic signal to an electric motor 236, controlling a calibrated airflow resistance control element 237 by means of gears 238 and 239. The acoustic horn shape of this example, along with its associated synthetic jet, is preferred, although one can envision other examples where the acoustic horn is not used. The main feature of these examples are, however, a calibrated airflow resistance control element 237, that controls the velocity and/or volume of airflow through the device. There exist many ways to achieve this calibrated airflow resistance control element, and one such way is way is with an inhalation threshold resistance valve which regulates airflow entering chamber 225 via inlet 228.

The airflow resistance valve assembly is comprised of a rotatable cap 240 with an integrally formed cylindrical wall slidably received through a cylindrical housing 241. Gear 239 is connected to, or forms the top of, rotatable cap 240. Gear 239, and/or the top of cap 240, contains one or more air inlet ports, shown as 242a and 242b, that allows airflow to enter airflow resistance control element 237, which allows airflow to enter chamber 225 via inlet 228, when this valve is open. Rotatable cap 240 also has a tubular guide 243 extending through it. The tubular guide has female threads 244 that is designed to receive the male threads of a thin rod 245. A load calibrated, coiled spring 246, or other resilient or biasing member, is positioned inside of the rotatable cap 240, around the tubular guide 243 and thin rod 245. A circular disc 247, along thin rod 245, is located within a chamber region 248, adjacent to reserve chamber 225, and serves as the actuator piston of threshold resistance valve 237. As spring 246 puts outward pressure on rotatable cap 240, circular disc 247 is pulled against the proximal surface of chamber 248, thereby blocking this chamber's proximal aperture 249.

Upon inhalation, when a threshold level of negative pressure, vacuum pressure, is applied on the valve assembly, the threshold valve will open as the spring compresses and the actuator piston moves away from its resting position. Cap 240 is able to slide within cylindrical housing 241, commensurate with gear 239 being able to slide along gear 238. When the threshold valve is open, ambient air enters the device through air inlets 242, and passes through chamber 248 and reserve chamber 225, entraining aerosolized particles, and carrying these particles out of the device through outlet 230. The threshold valve closes when negative pressure within chamber 225, and chamber 248, can no longer overcome the tension of the spring. The threshold valve 237 also serves as a calibrated airflow resistance control element. As electric motor 236 turns gears 238 and 239, cap 240 is rotated like a dial. When the cap is rotated, the distance that the thin rod 245 screws into the tubular guide 243 of the cap also changes, thereby affecting the space between the cap 240 and the cylindrical housing 241, and thus, the compression of the spring 246. By varying the tension of the spring, one can control inhalation resistance and the velocity of airflow through the device, which may allow for aerosol delivery with sustained maximal inspiration, inhalation. The number of partial or full revolutions that the electric motor 236 must spin in order to turn gears 238 and 239, and thus, cap 240, necessary to adjust the tension of load calibrated spring 246, is programmed into the digital control unit 218. Thus, digital control unit 218 can automatically adjust airflow resistance settings based on user inputs 219, or from data signals generated from airflow sensor 234. Other examples may utilize a manual means for adjusting calibrated airflow resistance settings.

The digital control unit 218 may also contain a microprocessor that can perform one or more functions, such as: providing an alarm function to signal when a treatment is due, a timer function to measure the duration of treatment and/or to turn off operation after a certain treatment duration, a counting function to determine the number of treatments, a function to keep track of the airflow resistance settings during treatment, a time/date function to track the treatments of one or more different medicament formulations, the ability to store settings for different medicament formulations, along with any other functions obvious to the use of this device. The digital control unit 218 may have an electronic speaker 250 that provides auditory feedback to the user regarding the user's progress and/or to adjust the user's inhalation rate or breathing pattern, and/or to provide the user with incentive. The electronic speaker may provide human sounding words to provide such auditory feedback, and may also voice aloud device settings and functions. The digital control unit may contain a USB port and/or memory card so that data can be interfaced with a computer or respiratory instrument.

This example utilizes a medicament strip with a single medicament blister. One can envision other examples where multiple blisters are housed on the strip, or a device that can hold and use multiple unit dosages of medicament, sequentially.

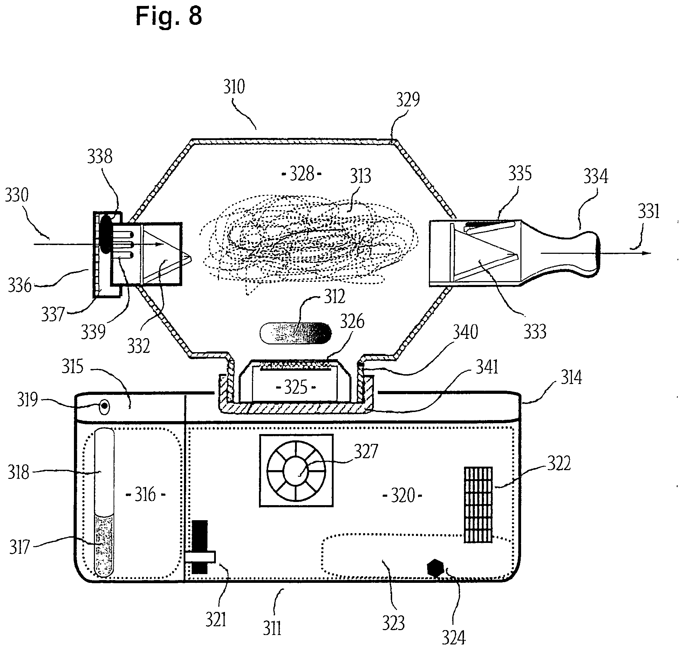

FIG. 8 shows a cross-section of a preferred embodiment example of an aerosol delivery device 310 that uses energy from a micro power source, such as a fuel cell 311, to vaporize a substance 312 to produce a condensation aerosol 313 for inhalation. Fuel cells are a type of electrochemical cell that generate direct current through oxidation of a fuel, usually in the presence of an electrolyte, without the danger of high-temperature combustion. Some example combinations of fuels and oxidants include: hydrogen and oxygen, and hydrocarbons and alcohols. Fuel cells also have an anode catalyst that breaks down fuel into electrons and ions. These ions are converted into waste chemicals, such as water or carbon dioxide, by the cathode catalyst. Like fuel cells, batteries also make use of electrochemical energy, but as they store this energy, and over time, batteries can lose their charge; whereas, fuel cells can be more reliable sources of energy, when stored for extended periods of time. Many fuel cells can achieve a higher energy density by volume, and a higher energy density by weight, than most lithium-ion battery alternatives. Fuel cell cartridges may be easily refillable or as disposable as a butane lighter. With a proton exchange membrane or polymer electrolyte membrane, PEM, direct methanol fuel cells are small and convenient for portable devices. For instance, direct methanol fuel cells utilize a water/methanol mixture as fuel, and oxygen, such as from ambient air, as an oxidant in the following oxidation-reduction reaction:
Half reaction at the anode:
   CH.sub.3OH+H.sub.2O.fwdarw.CO.sub.2+6H.sup.++6e.sup.-
Half reaction at the cathode:
   O.sub.2+4H.sup.++4e.sup.-.fwdarw.2H.sub.2O
The overall fuel cell reaction:
   CH.sub.3OH+1.5O.sub.2.fwdarw.CO.sub.2+2H.sub.2O

What follows is a description of the main components of fuel cell 311, which powers vaporizer 310. The main fuel cell housing 314 is adjoined to fuel cell cartridge 315, and in some embodiments may be removably attached. The cartridge contains a fuel reservoir 316 and a fuel 317, with a level or volume which can be viewed from a see-through window 318. The cartridge may also contain a fuel inlet 319 that allows the cartridge to be refilled, with a refueling device, not shown, as an alternative to being replaced when fuel is depleted. Within the fuel cell housing 314 is the reaction chamber 320, which contains all the components of a functional fuel cell, not shown, such as an anode and anode catalyst, a cathode and cathode catalyst, and an electrolyte, which may be a PEM. Fuel may be gravitationally fed into the reaction chamber 320 from the fuel reservoir 316, or other methods, such as capillary pressure or a micro pump, may be used. A switch, shunt, or actuator 321 controls the movement of fuel 317 into the reaction chamber 320, and essentially serves as a means to activate the fuel cell. In some embodiments, actuator 321 may be part of, or a lever to, a manual pump to draw fuel into the reaction chamber. Vent 322 allows an oxidant, such as oxygen from ambient air, to enter the reaction chamber 320. Emission reservoir 323 allows the, liquid, product(s) of the chemical reaction, such as water, to collect until released through emission plug or outlet 324. Gaseous product(s) of the chemical reaction, such as carbon dioxide, may also be released through a vent similar to, or the same as, vent 322.

Electrical energy produced by the fuel cell powers a heating element 325, which is a resistor. Heating element 325 has a heating surface 326, which may be metallic or ceramic, that can vaporize a substance 312, either by thermal conduction, and/or by thermal convection, to produce a condensation aerosol 313. In certain embodiments, heating element 325 may serve as an electrostatic charger able to produce an electrostatic charge in the substance, or particles thereof. Electrical energy between the fuel cell 311 and the heating element 325 is regulated by control element 327. Control element 327 may serve as a switch to turn the heating element on or off, and/or to turn the fuel cell on or off, or may serve as a dial to regulate the amount of electricity going to the heating element 325. In some embodiments, control element 327 is a thermostat that regulates the temperature of heating element 325, which may, or may not, be set by the user, and may include calibrated indicia. In other embodiments, control element 327 may consist of a pressure sensing or conductivity sensing lead, a touch panel, activated by direct user contact or touch. Still in other embodiments, control element 327 may consist of a timer that shuts off power to the heating element after a specified duration of time.

The condensation aerosol 313 that forms upon the cooling of a substance 312, after being vaporized, is contained within an aerosol, holding, chamber 328. Aerosol chamber 328 is comprised of a chamber housing 329, which has an air inlet end 330 and an air outlet end 331. The device may contain one-way valves 332 and 333, preferably duckbill valves, which trap the condensation aerosol within the chamber, until vacuum pressure, or a significant threshold vacuum pressure, generated from user inhalation, is able to open said one-way valves 332 and 333. In this manner, condensation aerosol is thusly contained in chamber 328 until airflow, originating at air inlet end 330, carries the aerosol through the device and out to the end user through the airflow outlet end 331 of the device. The airflow outlet end 331 of the device may contain a mouthpiece 334 that contours to the user's lips, allowing for an airtight seal. Said mouthpiece 334 may contain an exhaust port 335, comprised of an elastomeric one-way, flap, valve, which vents user exhalation, while one-way valve 333 prevents exhalation from entering the interior of the device. In alternative embodiments of this device, a mouthpiece may be attached to the airflow outlet end 331 via a long and/or flexible tube or hose.

This device also has a calibrated airflow resistance control element 336, which in this embodiment, consists of a user controlled airflow resistance dial 337 with one or more supplemental apertures 338. The user controlled airflow resistance dial 336 is flush with the airflow inlet end 330 of the device. Rotation of dial 337 aligns supplemental aperture(s) 338 with one or more airflow inlet passages 339, thereby controlling the amount of airflow allowed to enter the device and travel through these passages 339, having the affect of controlling the velocity and/or volume of airflow through the device. This, in effect, allows the user to adjust the dynamics of how the condensation aerosol is formed, entrained, and evacuated from the aerosol chamber, and may allow for aerosol delivery with a sustained maximal inspiration, inhalation.

In this embodiment, the housing 329 of aerosol chamber 328, and/or the heating element 325, and/or its surface 326, may be removably attached, so that substance residue can be removed from surface 326, and replaced by new substance. The substance may be in raw form, or may be contained or coated on a thin strip, wafer, pellet, or capsule. The contours of surface 326 may be designed to help hold the substance, and/or to help grind the substance into smaller pieces, making the substance more readily accessible for receiving heat. Aerosol chamber housing 329 may be removed for cleaning, as well. As such, aerosol chamber housing 329 may have a threaded base 340, that screws into a threaded base support 341 of the fuel cell housing 314.

Other embodiments of a micro power source may be envisioned for this device that utilize a fuel source to produce thermal heat, instead of, or in addition to, producing electricity as a fuel cell does. Other micro power sources may include a micro engine, micro-gas turbine, which may produce heat and electricity through combustion of a fuel that turns the turbine, or a micro heater that uses combustion or direct oxidation to release thermal energy. Such thermal energy may be applied to a heating surface, such as a radiator with a fan and/or air pump, to vaporize a substance using thermal conduction and/or convection currents. Other embodiments may use may utilize the electric energy produced by a fuel cell or micro-turbine to power a light or laser source, or a microwave source, to vaporize a substance with radiation. Additional other embodiments may include a cooling means by which the vapor produced is cooled more rapidly, such as having a water and/or ice cooling and/or conditioning means.

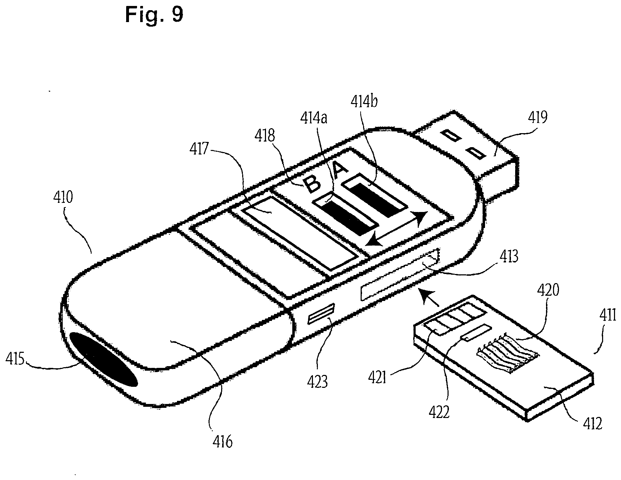

FIG. 9 shows a cross-section of yet another preferred embodiment example of an aerosol delivery device 410 according to the invention comprising a, replaceable/disposable, medicament cartridge or card 411, which may be shaped like a flash SD memory card commonly used in other electronic devices, such as computers and digital cameras. Medicament cartridge 411 has a housing 412, which may be asymmetrically shaped for proper orientation when inserted into the device through medication card slot 413. Air enters device 410 through one or more air inlet passages, shown in the diagram as 414a and 414b, and, aerosolized, air exits the device through at least one outlet 415, which the user can inhale from. As such, the distal end of the device has a region 416 that allows communication of the device with the user's lips during inhalation. In some embodiments, this mouthpiece region 416 may be removably attached to the device, so that it may be cleaned more efficiently. In the current embodiment, a slidable cover or panel 417 is selectively moveable, as shown by arrows, along the face of the device, and is able to, incrementally, cover one or more air inlets 414, and thus serves as an airflow resistance control element for controlling the velocity and/or volume of airflow into and/or through the device. This, in effect, allows the user to adjust the dynamics of how the condensation aerosol is formed, entrained, and/or evacuated from the aerosol device, and may allow for aerosol delivery with a sustained maximal inspiration, inhalation. Calibrated indicia 418 can be provided for this airflow control element, so the user can adjust airflow passages 414.

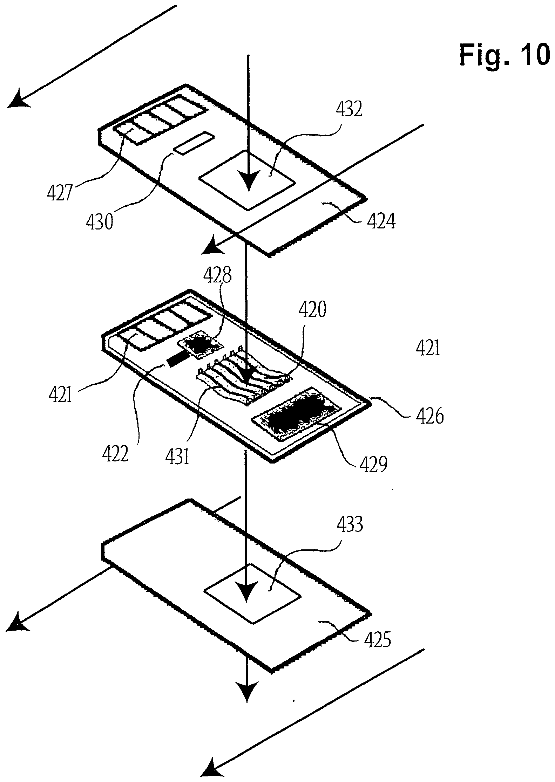

The inhaler device 410 may also have a USB connector or USB port 419, or micro USB connector or port, or the like, which can be used to send data or instructions between the inhaler and an external electronic device, such as a computer, respiratory instrument, or portable device, such as a smart phone, when the inhaler is connected to said electronic device, either directly or via a USB cable, or the like. The USB connector or port may also be used to draw electrical power from the external device, or an A/C adapter power cord, to recharge an internal battery, if present, and/or to power the circuitry of the inhaler, such as the circuitry of the medicament cartridge 411, including powering the vapor element 420. The vapor element 420 will be described in more detail in FIG. 10. Electrical contact pins 421 on the medicament card 411 allow for electrical communication between the card and the device. The medicament card may also have a selection switch or lock switch 422 that can regulate the use of the medicament card.

This embodiment may allow this device to serve as a smaller, more portable inhaler than other larger, more cumbersome device products, while having many advantages such as rapid onset, ease of use, and consistent dose and particle size. Moreover, the overall shape of this inhaler device embodiment can resemble the shape of a common USB flash drive, which may allow for greater user compliance as the inhaler device is less obtrusive in public. Such a portable inhaler can be stored or transported in a pocket, or connected via bracket 423 to a key chain, bracelet, necklace, or lanyard, not shown; thereby, allowing for greater convenience than larger vaporizer products, and making this type of inhaler suitable for both daily use and/or emergency situations. Dry medicament associated with vapor element 420, such as a lyophilized powder or other dry coatings, may also have the advantage of better storage and longer shelf life than other, liquid, formulations, and may have less issues with solubility and dependency on other reagents for formulation that generally limit other medicament applications.

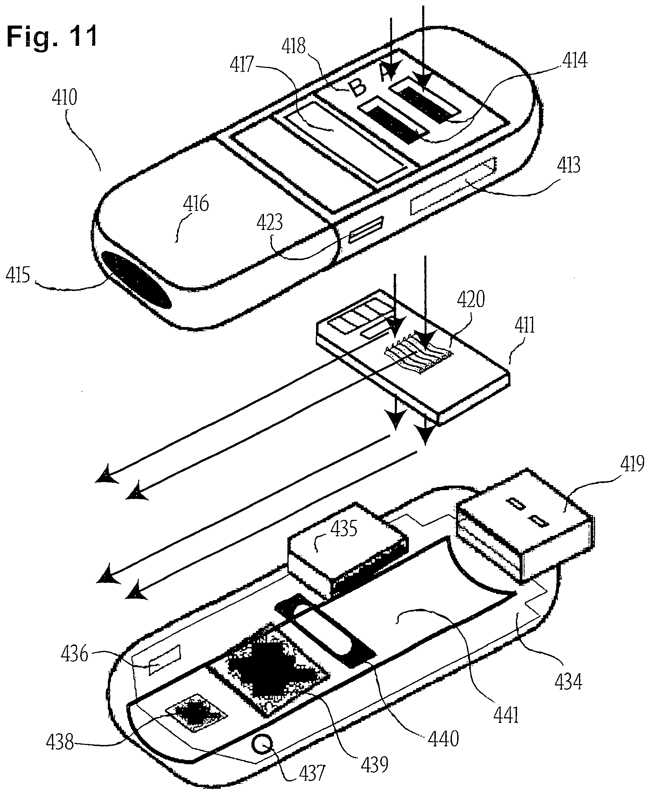

FIG. 10 shows an exploded view of the primary components of the medicament card 411, introduced in FIG. 9, and reveals the direction of airflow over, through, and around the medicament card, as indicated by arrows in the diagram. The medicament card has a first half housing 424 and a second half housing 425, that together form housing 412, with an internal circuit board 426, such as a printed circuit board or printed circuit assembly, therein. This circuit board 426 is connected to an arrangement of exterior facing, electrical contact pins 421 for communication with the device 410. Electrical contact pins 421 are exposed and may be further supported by contact window 427 of first half housing 424. Like a conventional memory card, the circuit board may include a microcontroller chip 428, such as an SD controller chip, and a flash memory chip 429, or electrically erasable programmable read-only memory device, for storing information, such as information regarding the type of medicament, dosage, and its use with the device. An optional selection switch or lock switch 422 of circuit board 426 is exposed through switch window 430 of the first half housing 424. As will next be described, the circuit board 426 also includes the vapor element 420 that produces a condensation aerosol, and whose activities are regulated by microcontroller chips 428 and/or 438, shown in FIG. 11. Controller chips can activate one or more components of the vapor element, either simultaneously, or sequentially, to release one or more dosages or dosage amounts. Controller chips may also regulate/adjust heating temperatures, power, and duration of heating, period, of the vapor element components. Flash memory chips 429 and/or 439, shown in FIG. 11, can be used for storing information, such as dosage counting, time/date memory, dosage usage history, serial number and medicament information, and a password to lockout unauthorized users. Furthermore, the flash memory may be preprogrammed and/or reprogrammed with instructions specific for the type of medicament, so the controller chip can regulate vapor element components according to the proper delivery characteristics of that particular medicament, or remaining dosages thereof, including proper vaporization settlings, such as temperature and duration, and perhaps even in accordance with the proper and/or selected airflow settings of the device.

In this embodiment, the vapor element comprises one or more electrically resistive heating supports, such as metal foil supports, or other means of conductive support, used to vaporize a substance to produce a condensation aerosol for inhalation. There exist numerous methods to apply the, medicinal, substance to the vapor element. For instance, the substance may be placed on, over, or in close proximity to the vapor element, such as in the format of a thin film. Such may be the case when the medicament card is reusable. If the medicament card is disposable, the substance may be coated, inkjet, printed, brushed on, or dried, using an evaporable solvent, on the vapor element directly, such as directly on its electrically resistive heating supports. The therapeutic application may consist of one or more different substances, dosage levels, and/or dosages. Therefore, the types of substances, dosages, and methods of applying the medicament to the vapor element, and the actual vapor element itself, including its material of construction, surface area, shape, thickness, thermal mass, and electrical properties, etc., are not intended to be limited in the scope of this patent. Other embodiments may be envisioned by which the vapor element may also serve as an electrostatic charger able to produce an electrostatic charge in the substance, or particles thereof.

Another component of vapor element 420, and its associated circuit board 426, are one or more spaces, holes, or vents 431 that allow air and/or vapor and/or aerosol to pass through so as to better suspend and entrain the substance particles in air, and to allow airflow to move the particles away from the vapor element, thereby, reducing subsequent deposition of such particles on the vapor element. Each half housing 424 and 425 of the medicament card housing 412 has a vapor element window 432 and 433, that can expose the vapor element 420, and/or its vents 431, and/or its released vapor, to airflow in device 410. Airflow passing through the medicament card is depicted by a series of vertical arrows on the diagram, while airflow passing over and under the medicament card are depicted as a series of diagonal arrows on the diagram.

When the vapor element, and/or controller, is activated by user input, such as from the detection of inhalation by an airflow sensor 436, shown in FIG. 11, one or more components of the vapor element rapidly heats, vaporizes, one or more substances into the gaseous phase. Airflow, such as generated from user inhalation, passes over, around, and/or through the medicament card to entrain and cool the vaporized substance into a condensation aerosol with a MMAD desirable for inhalation, such as for lower airway and/or deep lung aerosol delivery. The condensation aerosol is carried to the user by airflow traveling through the inhaler device.

The inhaler device may also have one or more airflow resistance control elements, which may be used to increase, maintain, and/or decrease airflow velocity and/or volume, and/or to keep the airflow velocity and/or volume within one or more desired ranges. As an example, slowing airflow velocity through the device can allow for the user to take a prolonged breath in, over several seconds, for sustained maximal inspiration, inhalation, while a series of heating elements are sequentially activated to vaporize and produce a condensation aerosol, rather continuously, over much of this same extended time course. Such a means of controlling airflow velocity, and/or while controlling aerosol generation, may enhance aerosol delivery efficiencies.

FIG. 11 is an exploded view of aerosol delivery device 410, with medicament card 411, as well as airflow arrows passing through the medicament card and/or device. From this exploded view, one can see that the device can also have a circuit board 434, such as a printed circuit board or printed circuit assembly. The circuit board contains a medicament card interface port or reader 435, which slidably receives the, asymmetrically shaped, medicament card 411, and contains electrical contact pins to communicate with the electrical contact pins 421 of the card. The medicament card reader 435 can transfer electrical signals, data and/or power, to and from the medicament card 411. Circuit board 434 may also contain a user input to activate the device, such as an airflow sensor 436 to activate the device when inhalation is detected, a LED indicator light 437 to signal when data is being transferred, or when the device is ready for use or is in use, a controller device or chip 438 that controls the activities of the circuit board, a flash memory chip 439, or electrically erasable programmable read-only memory device, for data storage and/or programming, and a crystal oscillator 440 that serves as an internal clock and may control data output. USB port or connector 419 is also on the circuit board to receive power, and/or transfer data between an external device, such as a computer or smart phone, and the aerosol delivery device with medicament card. The aerosol delivery device may also have its own power source, such as a rechargeable battery, not shown, which can receive current from an external device.

Lastly, this embodiment includes one or more airflow conduits 441 whereby aerosol is carried through the device and out to the user through outlet 415. The airflow conduit 441 may shield some of the electronic components of the device from the deposition of aerosols. The airflow conduit 441 may be made of anti-static materials, or have an anti-static coating, so that the conduit, itself, does not readily experience aerosol deposition. In other embodiments, this airflow conduit may be much more elaborate and serve additional purposes. For instance, in another embodiment, this airflow conduit is connected to the circuit board where it can receive energy to produce an electromagnetic force/field which may help repel aerosol particles from the conduit's walls, and/or may help control the velocity of or help move these aerosol particles through the device. Such a conduit may be ideal for delivering magnetically responsive nanoparticle aerosols, called nanomagnetosols. Nanomagnetosols have the potential for the enhanced targeting of aerosols to specific regions of the lungs when external magnets are used on the patient's chest.

As to the manner of usage and operation of the present invention, the same should be apparent from the above description. Accordingly, no further discussion relating to the manner of usage and operation will be provided.

With respect to the above description then, it is to be realized that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

## Claims

1. An aerosol delivery device (10; 210; 310; 410) comprising:
an airflow inlet (19; 242; 330; 414);
a mouthpiece region (416) having an outlet (20; 230; 331; 415), wherein the mouthpiece region is removably attached to the aerosol delivery device;
an airflow passage extending between the airflow inlet and the outlet;
a slot (413) to receive a cartridge, wherein the cartridge includes a substance placed over a vapor element, wherein the vapor element comprising an electrically-resistive heating support configured to, when activated, vaporize the substance to produce a condensation aerosol;
a battery to provide power;
a microcontroller chip (428; 438) coupled to the battery to receive power and configured to regulate the vapor element, wherein, in response to each inhalation, the microcontroller chip is configured to activate the vapor element to release a respective dose of the condensation aerosol of the substance; **characterised by**:
a USB connector or the like (419) configured to draw power from an external device or an A/C power adapter to charge the battery; and
an electrical contact configured to provide electrical communication between the microcontroller chip and the cartridge.

2. The aerosol delivery device of claim 1, wherein the USB connector or the like is further configured to send data or instructions to and receive data or instructions from an external electronic device.

3. The aerosol delivery device of claim 2, wherein the information includes, medicament information, a password, dosage counting, time/date memory, dosage usage history, serial number, instructions used by the microcontroller chip to regulate heating temperatures, power, a duration of heating, period, of the vapour element components.

4. The aerosol delivery device of claim 2, further comprising a memory chip configured to store at least some of the information.

5. The aerosol delivery device of claim 1, wherein the USB connector or the like includes a USB connector, a USB port, a micro USB connector, or a micro USB port.

6. The aerosol delivery device of claim 1, wherein the airflow passage further extends through the cartridge.

7. The aerosol delivery device of claim 1, further comprising a sensor configured to detect the inhalation and provide a signal to the controller indicating detection of the inhalation.

8. The aerosol delivery device of claim 1, wherein the microcontroller chip is configured to illuminate an indicator light when in use.

9. The aerosol delivery device of claim 1, wherein the substance includes at least one of a cannabinoid, tetrahydrocannabinol, a tetrahydrocannabinol salt, a tetrahydrocannabinol analogue, a tetrahydrocannabinol derivative, a tetrahydrocannabinol extract, or any combination thereof.

10. The aerosol delivery device of claim 1, wherein the substance includes at least one of nicotine, a nicotine salt, a nicotine analogue, a nicotine derivative, a nicotine extract, or any combination thereof.

11. The aerosol delivery device of claim 1, wherein the mouthpiece region is configured to be detachable from the aerosol delivery device.

12. The aerosol delivery device of claim 1, wherein the cartridge is configured to be replaceable/disposable.

13. The aerosol delivery device of claim 1, wherein the cartridge is configured to be reusable and refillable with the aerosolizable substance.

14. The aerosol delivery device of claim 1, further comprising a circuit board configured to include the microcontroller chip.

## Patentansprüche

1. Aerosolzufuhrvorrichtung (10; 210; 310; 410), die Folgendes umfasst:
einen Luftstromeinlass (19; 242; 330; 414);
eine Mundstückregion (416) mit einem Auslass (20; 230; 331; 415), wobei die Mundstückregion an der Aerosolzufuhrvorrichtung lösbar befestigt ist;
einen zwischen dem Luftstromeinlass und dem Auslass verlaufenden Luftstromkanal;
einen Schlitz (413) zum Aufnehmen einer Kartusche, wobei die Kartusche eine über einem Dampfelement platzierte Substanz einschließt, wobei das Dampfelement ein elektrisch widerstandsfähiges Erhitzungsträgerelement umfasst, wobei dieses konfiguriert ist, um bei einer Einschaltung die Substanz zu verdampfen, um ein Kondensationsaerosol herzustellen;
eine Batterie zum Bereitstellen von Leistung;
einen mit der Batterie gekoppelten Mikrocontrollerchip (428; 438) zum Aufnehmen von Leistung, der konfiguriert ist, um das Dampfelement zu regulieren, wobei der Mikrocontrollerchip konfiguriert ist, um als Reaktion auf jede Einatmung das Dampfelement einzuschalten, um eine jeweilige Dosis des Kondensationsaerosols der Substanz abzugeben; **gekennzeichnet durch**:
einen USB-Stecker oder dergleichen (419), der konfiguriert ist, um einer externen Vorrichtung oder einem AC-Netzadapter Leistung zu entnehmen, um die Batterie aufzuladen; und
einen elektrischen Kontakt, der konfiguriert ist, um elektrische Kommunikation zwischen dem Mikrocontrollerchip und der Kartusche bereitzustellen.

2. Aerosolzufuhrvorrichtung nach Anspruch 1, wobei der USB-Stecker oder dergleichen ferner konfiguriert ist, um Daten oder Befehle an eine externe elektronische Vorrichtung zu senden und Daten oder Befehle von der externen elektronischen Vorrichtung zu empfangen.

3. Aerosolzufuhrvorrichtung nach Anspruch 2, wobei die Informationen Arzneimittelinformationen, ein Passwort, eine Dosierzählung, einen Uhrzeit/Datum-Speicher, einen Dosiernutzungsverlauf, eine Seriennummer, Befehle, die vom Mikrocontrollerchip zum Regulieren der Heiztemperaturen, der Leistung, der Dauer oder Periode der Erhitzung der Dampfelementkomponenten genutzt werden, einschließen.

4. Aerosolzufuhrvorrichtung nach Anspruch 2, die ferner einen Speicherchip umfasst, der konfiguriert ist, um mindestens einige der Informationen zu speichern.

5. Aerosolzufuhrvorrichtung nach Anspruch 1, wobei der USB-Stecker oder dergleichen einen USB-Stecker, einen USB-Anschluss, einen Micro-USB-Stecker oder einen Micro-USB-Anschluss einschließt.

6. Aerosolzufuhrvorrichtung nach Anspruch 1, wobei der Luftstromkanal ferner durch die Kartusche verläuft.

7. Aerosolzufuhrvorrichtung nach Anspruch 1, die ferner einen Sensor umfasst, der konfiguriert ist, um die Einatmung zu detektieren und dem Controller ein die Detektion der Einatmung anzeigendes Signal bereitzustellen.

8. Aerosolzufuhrvorrichtung nach Anspruch 1, wobei der Mikrocontrollerchip konfiguriert ist, um eine Anzeigeleuchte bei der Nutzung aufleuchten zu lassen.

9. Aerosolzufuhrvorrichtung nach Anspruch 1, wobei die Substanz mindestens eines von einem Cannabinoid, einem Tetrahydrocannabinol, einem Tetrahydrocannabinolsalz, einem Tetrahydrocannabinolanalogon, einem Tetrahydrocannabinolderivat, einem Tetrahydrocannabinolextrakt oder einer beliebigen Kombination davon einschließt.

10. Aerosolzufuhrvorrichtung nach Anspruch 1, wobei die Substanz mindestens eines von Nikotin, einem Nikotinsalz, einem Nikotinanalogon, einem Nikotinderivat, einem Nikotinextrakt oder einer beliebigen Kombination davon einschließt.

11. Aerosolzufuhrvorrichtung nach Anspruch 1, wobei die Mundstückregion so konfiguriert ist, dass sie von der Aerosolzufuhrvorrichtung abnehmbar ist.

12. Aerosolzufuhrvorrichtung nach Anspruch 1, wobei die Kartusche so konfiguriert ist, dass sie austauschbar/als Einwegteil verwendbar ist.

13. Aerosolzufuhrvorrichtung nach Anspruch 1, wobei die Kartusche so konfiguriert ist, dass sie wiederverwendbar und mit der aerosolierbaren Substanz wiederbefüllbar ist.

14. Aerosolzufuhrvorrichtung nach Anspruch 1, die ferner eine Leiterplatte umfasst, die so konfiguriert ist, dass sie den Mikrocontrollerchip einschließt.

## Revendications

1. Dispositif de distribution d'aérosol (10 ; 210 ; 310 ; 410) comprenant :
une entrée de flux d'air (19 ; 242 ; 330 ; 414) ;
une région d'embout buccal (416) ayant une sortie (20 ; 230 ; 331 ; 415), la région d'embout buccal étant attachée de manière amovible au dispositif de distribution d'aérosol ;
un passage de flux d'air s'étendant entre l'entrée de flux d'air et la sortie ;
une fente (413) pour recevoir une cartouche, la cartouche incluant une substance placée sur un élément vapeur, l'élément vapeur comprenant un support chauffant électriquement résistif configuré pour, lorsqu'il est activé, vaporiser la substance afin de produire un aérosol de condensation ;
une batterie pour fournir une alimentation ;
une puce de microcontrôleur (428 ; 438) couplée à la batterie pour recevoir une alimentation et configurée pour réguler l'élément vapeur, en réponse à chaque inhalation, la puce de microcontrôleur étant configurée pour activer l'élément vapeur afin de libérer une dose respective de l'aérosol de condensation de la substance ;
**caractérisé par** :
un connecteur USB ou similaire (419) configuré pour tirer une alimentation d'un dispositif externe ou d'un adaptateur d'alimentation A/C pour charger la batterie ; et
un contact électrique configuré pour fournir une communication électrique entre la puce de microcontrôleur et la cartouche.

2. Dispositif de distribution d'aérosol selon la revendication 1, dans lequel le connecteur USB ou similaire est en outre configuré pour envoyer des données ou des instructions à et recevoir des données ou des instructions en provenance d'un dispositif électronique externe.

3. Dispositif de distribution d'aérosol selon la revendication 2, dans lequel les informations incluent, des informations de médicament, un mot de passe, un comptage de dosage, une mémoire de temps/date, un historique d'utilisation de dosage, un numéro de série, des instructions utilisées par la puce de microcontrôleur pour réguler des températures de chauffage, une alimentation, une durée de chauffage, une période, des composants d'élément de vapeur.

4. Dispositif de distribution d'aérosol selon la revendication 2, comprenant en outre une puce de mémoire configurée pour stocker au moins certaines des informations.

5. Dispositif de distribution d'aérosol selon la revendication 1, dans lequel le connecteur USB ou similaire inclut un connecteur USB, un port USB, un connecteur micro-USB, ou un port micro-USB.

6. Dispositif de distribution d'aérosol selon la revendication 1, dans lequel le passage de flux d'air s'étend en outre à travers la cartouche.

7. Dispositif de distribution d'aérosol selon la revendication 1, comprenant en outre un capteur configuré pour détecter l'inhalation et fournir un signal au dispositif de commande indiquant la détection de l'inhalation.

8. Dispositif de distribution d'aérosol selon la revendication 1, dans lequel la puce de microcontrôleur est configurée pour illuminer un voyant lumineux lors de l'utilisation.

9. Dispositif de distribution d'aérosol selon la revendication 1, dans lequel la substance inclut au moins un élément parmi un cannabinoïde, du tétrahydrocannabinol, un sel de tétrahydrocannabinol, un analogue de tétrahydrocannabinol, un dérivé de tétrahydrocannabinol, un extrait de tétrahydrocannabinol, ou une combinaison quelconque de ceux-ci.

10. Dispositif de distribution d'aérosol selon la revendication 1, dans lequel la substance inclut au moins un élément parmi de la nicotine, un sel de nicotine, un analogue de nicotine, un dérivé de nicotine, un extrait de nicotine, ou une combinaison quelconque de ceux-ci.

11. Dispositif de distribution d'aérosol selon la revendication 1, dans lequel la région d'embout buccal est configurée pour être détachable du dispositif de distribution d'aérosol.

12. Dispositif de distribution d'aérosol selon la revendication 1, dans lequel la cartouche est configurée pour être remplaçable/jetable.

13. Dispositif de distribution d'aérosol selon la revendication 1, dans lequel la cartouche est configurée pour être réutilisable et reremplissable avec la substance aérosolisable.

14. Dispositif de distribution d'aérosol selon la revendication 1, comprenant en outre une carte de circuit imprimé configurée pour inclure la puce de microcontrôleur.
